# EUROPEAN PATENT APPLICATION

(11) **EP 2 315 032 A1**
(43) Date of publication of application: **27.04.2011**
(21) Application number: 09382220.3
(22) Date of filing: 20.10.2009
(51) Int. Cl.: G01N 33/68

(54) **3-Nitrotyrosinated fibrinogen as a diagnostic marker in ischemic stroke and uses thereof**

(71) Applicant: Universitat Pompeu-Fabra, 08002 Barcelona (ES)
(72) Inventor: Ill Raga, Gerard, 08013 Barcelona (ES); Muñoz López, Francisco José, 08024 Barcelona (ES); Valverde de Castro, Miguel Angel, 08912 Badalona (Barcelona) (ES)
(74) Representative: ZBM Patents

(57) **Abstract**

An in vitro method for diagnosing an ischemic stroke in a patient, comprising measuring the amount of 3-nitrotyrosinated fibrinogen in the patient, which further comprises comparing the obtained value with known value(s) associated with an ischemic stroke and/or control value(s). And also a method of deciding or recommending whether to iniciate pharmacological treatment of a patient suspected of suffering an ischemic stroke which method comprises: a) measuring, in vitro, the amount of 3-nitrotyrosinated fibrinogen in the patient; b) diagnosing the ischemic stroke by comparing the value obtained in step (a) with known value(s) associated with an ischemic stroke and/or control value(s), c) wherein, ca) if the patient is diagnosed of suffering an ischemic stroke, then the initiation of the treatment is recommended, and/or cb) if the patient is diagnosed of not suffering an ischemic stroke, then refraining from the treatment is recommended, optionally in consideration of the result of an examination of the patient by a neurologist.

## Description

### FIELD OF THE INVENTION

The present invention provides methods and markers for the diagnosis of ischemic strokes.

### BACKGROUND ART

Stroke is a leading cause of death and disability worldwide and still nowadays there is not a rapid and accurate biomarker for the diagnosis of stroke. A stroke is a pathological disruption in the brain blood supply that yields to the loss of brain function(s). It can be due to ischemia (lack of blood supply), caused by thrombosis or embolism, or due to a hemorrhage.

The so called ischemic strokes represent approximately 75% of the total of strokes. There are several types of ischemic strokes based on the cause of the ischemic process: (1) atherothrombotic stroke, when the obstruction of the blood supply is due to an atheroma from elsewhere in the body; (2) cardioembolic stroke, when the obstruction of the blood supply is due to an embolus of cardiac origin; (3) lacunar stroke, which is a type of stroke that results from occlusion of the penetrating arteries that provide blood to the brains's deep structures; and finally (4) undetermined stroke or stroke without an obvious explanation.

The effects of an ischemic stroke depend on which part of the brain is injured, and how severely it is injured. It may cause several physical symptoms, i.e. sudden weakness, loss of sensation, difficulty to speak and headache, among others. But sometimes, ischemic strokes could be completely painless. It is, hence, very important to recognise the warnings signs of them and to get immediate medical attention if they occur. A late diagnosis of an ischemic stroke results in systemic damages such as hemiplegia, in other words, hindering normal movement of half of the body, inability to walk, swallow or even to speech. A rapid and accurate diagnosis of an accute ischemic stroke would help to shorten the mortality and to reduce systemic damages associated with its late diagnosis.

In ischemic processes, and, hence, in an ischemic stroke, nitric oxid (NO) is produced. NO is a molecule with pleiotropic effects in different tissues, but its major systemic function is to induce vasodilatation. After the ischemic event takes place there is a burst in the production of free radicals such as superoxide anion (O₂·-). NO combines chemically with O₂·- to deliver the highly reactive peroxynitrite anion (ONOO-). ONOO- nitrates proteins, an irreversibly chemical process consisting in the addition of a nitro group (NO₂) into tyrosine residues to generate 3-nitrotyrosine. Such process is also known as nitrotyrosination. The toxic effects of ONOO- extend to different cellular elements and also plasmatic circulating proteins, especially fibrinogen. Fibrinogen is the third most abundant plasma protein representing 2-3% of the circulating proteins and is the final responsible for blood coagulation.

Vadseth et al. "Pro-thrombotic state induced by post-translational modification of fibrinogen by reactive nitrogen species" J. Biol. Chem. (2004) 279:8820-8826, quantified the circulating levels of nitrotyrosined fibrinogen in the plasma of patients with documented coronary artery disease, which is an ischemic process. The authors proposed an association between nitrotyrosinated fibrinogen and an increase in the incidence of adverse effects in coronary artery disease.

Notwithstanding the above physiological knowledge of ischemic processes, no advance has been done to develop more rapid methods for the rapid and accurate diagnosis of an acute ischemic stroke event based on it. A biomarker for the detection of an ischemic stroke in acute phase has been proposed as extremely useful. However, so far none of the proposed biomarkers has been neither specific nor sufficient to determine the presence of an acute ischemic stroke (Hill et al. "Diagnostic biomarkers for stroke: a stroke neurologist's perspective" Clinical Chemistry (2005) 51 (11): 2001-2002; Whiteley et al. "Blood biomarkers in the diagnosis of ischemic stroke. A systematic review" Stroke (2008) 39:2902-2909).

Current state of the art in the diagnosis of stroke involves a physical examination and imaging procedures such as computed tomography (CT) scan, angiogram, magnetic resonance imaging (MRI), single photon emission computed tomography (SPECT) and positron emission tomography (PET). A stroke diagnosis based on CT scan, SPECT, PET and MRI cannot be used for a rapid, whithin few hours diagnosis of stroke. While physical examination is rapid, it can only detect large strokes. Moreover, any clinical neurological screening test will be limited by the training and experience of the medical examiner. Trombolytic therapy has been proven to be effective for the treatment of acute ischemic stroke. It is critical to restore proper blood flow to the brain as soon as possible to prevent tissue damage, rapid diagnosis of stroke is critical to the survival of the patient and the minimisation of any effects of the stroke to the patient. If treated after occurrence of the ischemic event, most stroke patients can expect full or partial recovery. Preferably, the patient should be treated up to 24 hours after the event, more preferably up to 3 hours and most preferably up to 1 hour.

In summary, because stroke occurs rapidly and requires immediate treatment, a rapid and accurate diagnosis of an ischemic stroke that allows to proceed with the appropiate treatment is required. This suggests the need for an adjunctive test (biomarker) that can provide diagnostic information above and beyond routinary screening clinical exams.

### SUMMARY OF THE INVENTION

The present invention encompasses methods useful in the diagnosis and treatment of stroke, particularly ischemic stroke.

The present invention fulfils a long-felt need, namely a rapid and accurate diagnosis of an ischemic stroke using a biomarker.

The present invention is based on the unexpected inventors' finding that the plasmatic fibrinogen is significantly 3-nitrotyrosinated (up to 50 times above the control values) up to 24 hours after an ischemic stroke event.

The present invention, hence, provides simple, inexpensive and novel method for diagnosing an ischemic stroke, comprising measuring the amount of 3-nitrotyrosinated fibrinogen in a patient. In summary, this is the first time that a rapid and accurate method for the detection of an ischemic stroke is disclosed.

The event of nitrotyrosination of fibrinogen in patients with documented coronary artery disease has been disclosed by Vadseth *et al.* These authors report a selective increase in circulating levels of nitrotirosinated fibrinogen (called nitrated fibrinogen in Vadseth *et al.)* in patients with coronary artery disease. Therefore, Vadseth *et al.* teaches the relationship between the levels of nytrotirosinated fibrinogen and cardiac diseases.

In contrast to the teaching of Vadseth *et al.,* the present invention relates to the determination of the level of 3-nitrotyrosinated fibrinogen in a sample taken from a subject for the diagnosis of an ischemic stroke.

It is well-known for the person skilled in the art that cardiovascular diseases and ischemic strokes are two completely different clinical entities.

During the ischemic stroke, and as result of the ischemic event itself, there is an occlusion of the cerebral blood vessels. In view of this, one would expect a blockade in the systemic distribution of any biomarker produced during the ischemic stroke.

However, the inventors have found that the volemic distribution of 3-nitrotyrosined fibrinogen allows its significant detection immediately after the ischemic event and up to 24 hours, being increased its concentration up to 50 times above the control values. The surprisingly and unexpectedly high levels of 3-nitrotyrosined fibrinogen observed after an ischemic stroke accident would imply that all the cerebral vasculature enters into reactivity in order to produce NO and induce the reperfusion of the cerebral.

Another object of the present invention is also a method of deciding or recommending whether to iniciate pharmacological treatment of a patient suspected of suffering an ischemic stroke which method comprises: a) measuring, in vitro, the amount of 3-nitrotyrosinated fibrinogen in the patient; b) diagnosing the ischemic stroke by comparing the value obtained in step (a) with known value(s) associated with an ischemic stroke and/or control value; c) wherein, ca) if the patient is diagnosed of suffering an ischemic stroke, then the initiation of the treatment is recommended, and/or cb) if the patient is diagnosed of not suffering an ischemic stroke, then refraining from the treatment is recommended, optionally in consideration of the result of an examination of the patient by a neurologist.

It is also an object of the present invention the use of 3-nytrotirosinated fibrinogen as a clinical diagnostic marker for ischemic stroke.

Another object of the underlying invention is the use of 3-nytrotirosinated fibrinogen as a therapeutical marker.

It is also part of the present invention the use of diagnostic means capable of measuring a patient's amount of 3-nitrotyrosinated fibrinogen in any of the methods disclosed herein.

The present invention is, hence, particularly advantageous to identify patients suffering an ischemic stroke, and, hence, differentiate between patients with hemorrhagic and ischemic stroke; and also to decide whether to effectively treat them.

### BRIEF DESCRIPTION OF THE DRAWINGS

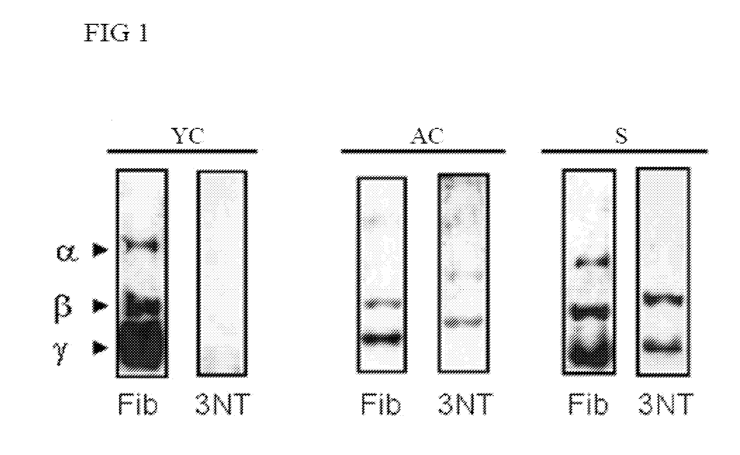

Figure 1. Representative western blots showing the extent of nitrotyrosination in fibrinogen immunoprecipitated from a young healthy control individual (left pannels), an aged control individual (middle pannels) and an ischemic stroke patient (right pannels); YC=Young Control; AC=Aged Control; S=Stroke; Fib=Fibrinogen; 3NT=3-nitrotyrosinated fibrinogen. This figure refers to Example 1.

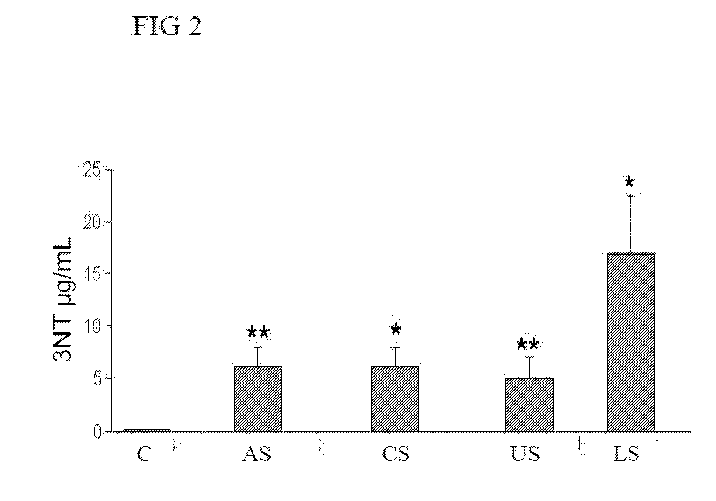

Figure 2. Extent of fibrinogen nitrotyrosination in controls and atherotrombotic, cardioembolic, undetermined and lacunar stroke patients. Data are mean ± Standard Error of Mean (SEM). * corresponds to a p value < 0.01; ** corresponds to a p value < 0.005. N-Tyr=3-nitrotyrosinated fibrinogen; C =Controls; AS=Atherothrombotic Stroke; CS=Cardioembolic Stroke; US=Undetermined Stroke; LS=Lacunar Stroke. This figure refers to example 2.

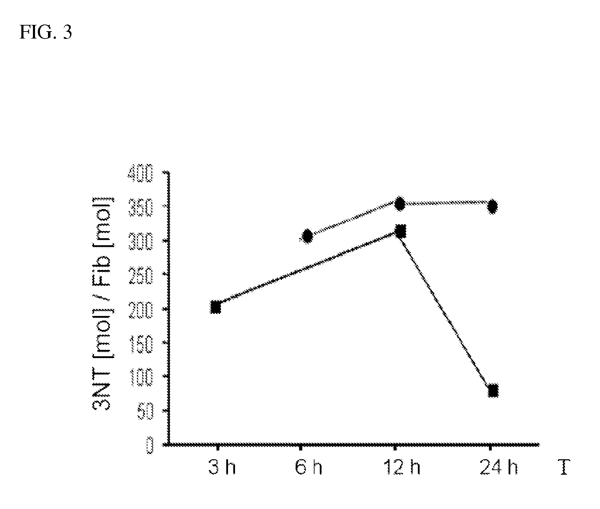

Figure 3. Time course evolution of fibrinogen nitrotyrosination, expressed as moles of 3-nitrotyrosinated fibrinogen/moles of normal fibrinogen, in two human patients interned at the hospital 3h (lower line) or 6h (upper line) after having suffered an ischemic stroke; 3NT=3-nitrotyrosinated fibrinogen. This figure refers to Example 2.

### Definitions

In general, the following words or phrases have the indicated definition when used in the description, examples ad claims.

The term "diagnosis" is known to the person skilled in the art. Diagnosing is understood as becoming aware of a particular medical condition, disease, complication or risk.

The term "patient", according to the present invention, relates to a healthy individual, an apparently healthy individual or particularly an individual suffering from a disease. The patient can be both a male or a female individual, particularly the patient is suffering from an ischemic stroke.

In the methods of the present invention, the "test sample" refers to a sample of bodily fluid obtained for the purpose of diagnosis, prognosis, or evaluation of a subject of interest, such as a patient. In certain embodiments, such a sample may be obtained for the purpose of determining the outcome of an ongoing condition or to determine whether a pharmacological treatment should be applied to a subject.

The term "level" relates to amount or concentration of nitrotyrosinated fibrinogen in a sample taken from a patient or subject (e.g. plasma). The term "measuring" according to the present invention relates to determining the amount or concentration, preferably semiquantitatively or quantitatively of the nitrotyrosinated fibrinogen.

The term "control level" or "control value" as used in the state of the art and also herein refers to the amount of a marker obtained in the sample obtained from a control subject(s), in other words, an ischemic stroke-free subject(s).

The term "therapeutical marker" as used herein, refers to nitrotyrosinated fibrinogen to be used as target for screening test samples obtained from subjects to establish whether said subject suspected of suffering a disease is within the therapeutical window in which a pharmaceutical treatment if applied is effective.

### DETAILED DESCRIPTION OF THE INVENTION

Fibrinogen is the initial agent of clot formation and is one of the therapeutic targets in stroke. The inventors have found that fibrinogen is nitrotyrosinated at very early moments after the ischemic stroke event (see Table 1) and the levels of nitrotyrosination are maintained up to 24 h after the ischemia onset.

Based on the above unexpected effect, the present invention provides simple, inexpensive and novel methods for diagnosing an ischemic stroke. One embodiment of the present invention is, hence, directed to an in vitro method for diagnosing an ischemic stroke in a patient, comprising measuring the amount of 3-nitrotyrosinated fibrinogen. Illustrative non-limitative examples of test samples include blood, serum and plasma. In a preferred embodiment of the first aspect of the invention, the test sample is plasma.

Any procedure known in the art for the measurement of the analyte can be used in the methods of the underlying invention. Such procedures include but are not limited to the spectrophotometrically determination of the stable product 3-nitrotyrosine or immunodetection with anti-3-nitrotyrosine antibodies and western blot. In a preferred embodiment of the present invention 3-nitrotyrosine is determined spectophotometrically by the absorbance measurement at 420 nm (ph>9).

The method of the present invention further comprises comparing the obtained value with known value(s) associated with an ischemic stroke and/or control value(s). In a more detailed description of the methods of the underlying invention, the total amount of 3-nitrotyrosinated fibrinogen determined in a sample taken from a patient is compared to (1) the control value(s), which is the amount of 3-nitrotyrosinated fibrinogen in a similar sample(s) from normal subject(s), and/or (2) to known value(s) of patient(s) with ischemic stroke. One of the ordinary skilled in the art will readily establish the appropiate control value(s) as well as the value(s) associated with an ischemic stroke event to be used in the methods of the present invention without undue experimentation. In case that the amount of 3-nitrotyrosinated fibrinogen in the sample taken from a subject is similar to control value(s) and/or the known value(s) of patient(s) with ischemic stroke, the subject from which the sample is derived is classified as suffering an ischemic stroke.

Guidance as to what 3-nitrotyrosinated fibrinogen values are associated with the occurrence of an acute ischemic stroke event following the methods of the present invention can be derived from those values disclosed in Table 1. It is evident that the values given in said Table 1 serve as a first reference. The person skilled in the art is able to know how to determine the values to be used as reference values in the methods of the underlying invention.

For example, the so called 3-nitrotyrosinated fibrinogen control value is between 0 and 0.15 µg/mL plasma. Examples of values associated with an ichemic stroke are disclosed in Table 1. For example, the 3-nitrotyrosinated fibrinogen mean value of subjects suffering from an atherothrombotic stroke was 38.5 fold higher than the corresponding value in controls; the 3-nitrotyrosinated fibrinogen mean value of subjects suffering from cardioembolic was 38.6 fold higher than the corresponding value in controls; the 3-nitrotyrosinated fibrinogen mean value of subjects suffering from an undetermined stroke was 31.8 fold higher than the corresponding value in controls; and finally, the 3-nitrotyrosinated fibrinogen mean value of subjects suffering from lacunar strokes was 105 fold higher than the corresponding value in controls.

Definitive therapy of an ischemic stroke are directed to avoid the presence of clots since the more rapidly bloodflow is restored to the brain, the fewer brain cells die. Thus, stroke patients are treated with thrombolytic agents to break the clot, antiplatelet anticoagulant to avoid the aggregation of platelets or by trombectomy to remove the clot. Therefore, pharmacologic thrombolysis is an essential effective treatment during the first hours after the ischemic stroke. Said treatment comprises administering recombinant tissue plasminogen activator (rt-PA); the fibrinolytic action of rt-PA is beneficial within the first hours after the onset of ischemic stroke, preferably within the first three hours. Furthermore, high doses of antiplatelet anticoagulant, i.e. acetylsalicylic acid can be given within 48 hours after the onset of the ischemic stroke. Other thrombolitic agents to break the clot and antiplatelet anticoagulants are known to the person skilled in the art and also belong to the present invention. Finally, regarding treatment of an ischemic stroke, since fibrinogen nitrotyrosination could be contributing to the worsening of the stroke effects, the inventors tested the effect of reduced glutathione (GSH), a well-known physiological antioxidant, in fibrinogen nitrotyrosination. GSH demonstrated to avoid it, opening the possibility for the treatment with antioxidants immediately after an ischemic stroke occurs.

In view of the above, the present invention also concerns a method of deciding or recommending whether to iniciate pharmacological treatment of a patient suspected of suffering an ischemic stroke which method comprises: a) measuring, in vitro, the amount of 3-nitrotyrosinated fibrinogen in the patient; b) diagnosing the ischemic stroke by comparing the value obtained in step (a) with known value(s) associated with an ischemic stroke and/or control value(s); c) wherein, ca) if the patient is diagnosed of suffering an ischemic stroke, then the initiation of the treatment is recommended, and/or cb) if the patient is diagnosed of not suffering an ischemic stroke, then refraining from the treatment is recommended, optionally in consideration of the result of an examination of the patient by a neurologist.

In a preferred embodiment, the above method is carried out for deciding or recommending a pharmacological treatment selected from the following ones trombolytic, antiplatelet anticoagulant and antioxidants. In a more preferred embodiment of the present invention the trombolytic treatment used is rt-PA. In another preferred embodiment the antiplatelet anticoagulant treatment used is acetylsalicylic acid. In stilll another preferred ambodiment, the antioxidant treatment used is GSH.

The present invention also comprises the use of 3-nytrotirosinated fibrinogen as a clinical diagnostic marker for ischemic stroke and also the use of 3-nytrotirosinated fibrinogen as a therapeutical marker. In a preferred embodiment, the therapeutical treatment is selected from the following ones trombolytic, antiplatelet anticoagulant and antioxidants.

Diagnosis according to the present invention is preferably done by use of a diagnostic means. A diagnostic means is any means that allows to measure the amount, or concentration of a substance of interest, particularly 3-nitrotyrosinated fibrinogen. Reagents or means for detecting 3-nitrotyrosinated fibrinogen could be comprised in kits for carrying out the methods of the present invention. It is, hence, another object of the present inventuion the use of diagnostic means capable of measuring a patient's level of 3-nitrotyrosinated fibrinogen in any of the methods disclosed in the underlying invention.

The methods of the present invention can be use alone or in conjuction with known test in the art for diagnosing an ischemic stroke.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1. Extent of nitrotyrosination in fibrinogen immunoprecipitated from a young healthy control, an aged healthy control and a stroke patient

To study the relevance of our findings in stroke the inventors immunoprecipitated fibrinogen from human plasma belonging to three different individuals: a young control, an aged control (prone to have certain levels of oxidative stress) and an ischemic stroke patient.

Plasma samples were lysed on ice with a solution containing 1 M Tris-HCl, 1% Nonidet P-40, 150 mM NaCl, 5 mM EDTA, 1 mM sodium orthovanadate, 1 mM dithiotreitol, pH 7.4 and a protease inhibitor cocktail (Roche). Protein concentration was determined by a Bio-Rad protein assay. Protein samples were analyzed by using 10% Tris-glycine gels for fibrinogen nitrotyrosination. Gels were ran at 150 V for 1 h and transferred to nitrocellulose membranes (Millipore) at 100 V for 2 h. Membranes were blocked in Tween 20-Tris buffer solution (100 mM Tris-HCl, 150 mM NaCl, pH 7.5), containing 5% milk, and incubated for 2 h at 25°C with 1:1,000 rabbit anti-nitrotyrosine polyclonal Ab (Invitrogen) or rabbit polyclonal anti-fibrinogen Ab (Dako). Peroxidase-conjugated donkey anti-rabbit Abs (Amersham Bioscience) was used as secondary Ab at 1:5,000 for 1 h at 25°C. Bands were visualized with Super Signal (Pierce) and Amersham Bioscience Hyperfilm ECL kit.

The three different fibrinogen samples were analyzed by western blot using anti-nitrotyrosine and anti-fibrinogen antibodies. Nitrotyrosinated fibrinogen was comparatively high in the stroke patient whereas the signal was very low in the aged control and inexistent in the young control (see also figure 1).

### Example 2. Quantification of the 3-nitrotyrosinated fibrinogen as clinical diagnostic marker of an ischemic stroke

Human plasma samples were supplied by the Servei de Neurologia (Hospital del Mar). The procedure was approved by the ethic committee of the lnstitut Municipal d'investigacions Mèdiques-Universitat Pompeu Fabra. Human plasma samples were obtained from patients at the very early moments (around three hours) atherothrombotic (17 individuals; 70.5 ± 2.6 years old; 3NT-Fib was 38.5 fold higher than controls), cardioembolic (14 individuals; 78.1 ± 2.4 years old; 3NT-Fib was 38.6 fold higher than controls), undetermined (8 individuals; 73.8± 4.4 years old; 3NT-Fib was 31.8 fold higher than controls) and lacunar strokes (12 individuals; 67.1 ± 3.9 years old; 3NT-Fib was 105 fold higher than controls) and controls (13 individuals; 41.6 ± 5.9 years old).

Three hundred and fifty µL of human plasma samples were exposed to 5 µg of anti-human fibrinogen polyclonal Ab (Dako). Samples were shaken overnight at 4°C. After adding 20 µg of protein G-sepharose (Sigma) they were shaken for 2 h at 25°C. Subsequent centrifugations at 10.000 r.p.m. for 10 min and three washes with PBS were carried out. Immunoprecipitated proteins were split up from protein G and antibody by boiling samples at 100°C for 6 min.

Three-Nitrotyrosine was determined spectrophotometrically by the absorbance measurement at 420 nm (pH>9). Protein concentration in the fibrinogen samples resulting from immunoprecipitation was determined, and 44 mg of fibrinogen was diluted to 100 mL of basic PBS (pH=12). Absorbance was measured at 415 nM. A calibration curve using serial dilutions of free 3-nitrotyrosine (Sigma) was carried out to quantify the nitrotyrosination of the fibrinogen samples (µg/mL plasma).

The results of the quantification of the of 3-nitrotyrosinated fibrinogen in the plasma of the subjects selected suffering from a stroke are presented in Table 1 below:

**TABLE 1**

| | **3NT µg/mL** | | | **Age** | | | |
|---|---|---|---|---|---|---|---|
| | **Media** | **SEM** | **n** | **mean** | **SEM** | **males** | **females** |
| **Controls** | 0,15965433 | 0,08784413 | 13 | 41,6923077 | 5,96075264 | 10 | 3 |
| **Aterotrombotic** | 6,17971111 | 1,71289576 | 17 | 70,5294118 | 2,69956103 | 8 | 9 |
| **Cardioembolic** | 6,04848645 | 1,85142973 | 14 | 78,1428571 | 2,41300766 | 5 | 9 |
| **Indetermined** | 5,09726298 | 1,86917825 | 8 | 73,8571429 | 4,40846048 | 2 | 6 |
| **Lacunar** | 16,8082819 | 5,6734545 | 12 | 67,1818182 | 3,97454019 | 7 | 5 |

With these experiments, we addressed the relationship between fibrinogen nitrotyrosination and the different types of ischemic stroke. The presence of 3-nitrotyrosinated fibrinogen was very low in controls (0.15 ± 0.08 µg/mL).

Interestingly, the values of 3-nitrotyrosinated fibrinogen in patients were significantly higher in comparison to controls for atherothrombotic (p<0.005), cardioembolic (p<0.01), undetermined (p<0.005) and lacunar (p<0.01) strokes. In other words, the levels of 3-nitrotyrosinated fibrinogen in the plasma of subjects with all 3 types of stroke and those with undetermined stroke were indicative of an accute ischemic stroke (see also figure 2). Following the above methodology, a time course up to 24 h was also performed with two patients having an ischemic stroke. The plasma levels of 3-nitrotyrosinated fibrinogen was quantified at different time points during the acute phase of the ischemic stroke, namely after 3 h, 6 h, 12 h and 24 h. A maximum nitrotyrosination peak appears 12 h after the stroke in both patients (see figure 3).

### Example 3. Nitrotyrosination of plasmatic fibrinogen in an in vivo animal model of ischemia

Animals were Sprague-Dawley rats (three month old; males; n=76) from lffa-Credo. Rats were anesthetised with 4 to 1.5% halothane to insert the stainless steel cannulas in the right lateral ventricle the day before ischemia. In a group of rats, a truncated 21 G needle was implanted in a burr hole to measure cortical perfusion (CP). Both cannulas and needles were fixed to the skull with two miniature screws and dental cement. The wound was sutured and the animal was allowed to recover for 24 h. Focal cerebral ischemia was produced by transient intraluminal occlusion of the MCA, as previously reported (Justicia C. et al. "Anti-VCAM-1 antibodies did not protect against ischemic damage either in rats or in mice" J. Cereb. Blood Flow Metab. (2006) 26(3):421-32). After surgery, rats were allowed to recover spontaneous breathing and were kept in their cages with free access to food and water. Five hundred mL of plasma samples were extracted from rats before the experimental ischemia was set (0 h) and at 3, 6, 12 and 24 h after the ischemia for posterior fibrinogen analysis. After collection of last plasma sample, rats were killed while under halothane anesthesia, and the brains were rapidly frozen and kept at -20°C.

Fibrinogen was immunoprecipitated from plasma and the extent of nitrotyrosination was determined as follows: 350 µL of rat plasma samples were exposed to 5 mg of anti-rat fibrinogen polyclonal antibody (Accu-Specs). Samples were shaken overnight at 4°C. After adding 20 mg of protein G-sepharose (Sigma) they were shaken for 2 h at 25°C. Subsequent centrifugations at 10.000 r.p.m. for 10 min and three washes with PBS were carried out. Immunoprecipitated proteins were split up from protein G and antibody by boiling samples at 100°C for 6 min.

The present inventors found a dramatic increase in fibrinogen nitrotyrosination with a peak at 3 h. High levels of nitrotyrosination were detected up to 24 h demonstrating that fibrinogen nitrotyrosination was maintained as a consequence of brain ischemia along the time (data not shown).

## Claims

1. An in vitro method for diagnosing an ischemic stroke in a patient, comprising measuring the amount of 3-nitrotyrosinated fibrinogen in the patient.

2. The method of claim 1, which further comprises comparing the obtained value with known value(s) associated with an ischemic stroke and/or control value(s).

3. A method of deciding or recommending whether to iniciate pharmacological treatment of a patient suspected of suffering an ischemic stroke which method comprises:
a) measuring, in vitro, the amount of 3-nitrotyrosinated fibrinogen in the patient,
b) diagnosing the ischemic stroke by comparing the value obtained in step (a) with known value(s) associated with an ischemic stroke and/or control value(s),
c) wherein,
ca) if the patient is diagnosed of suffering an ischemic stroke, then the initiation of the treatment is recommended, and/or
cb) if the patient is diagnosed of not suffering an ischemic stroke, then refraining from the treatment is recommended,
optionally in consideration of the result of an examination of the patient by a neurologist.

4. The metod of claim 3, wherein the pharmacological treatment is selected from the following ones trombolytic, anticoagulant and antioxidants.

5. The method of claim 4, wherein the trombolytic used is recombinant tissue plasminogen activator.

6. The method of claim 4, wherein the anticoagulant used is acetylsalicylic acid.

7. The method of claim 3 wherein the antioxidant used is reduced glutathione.

8. The method of any of the claims 1 to 7, wherein the sample is plasma.

9. Use of 3-nytrotirosinated fibrinogen as a clinical diagnostic marker for ischemic stroke.

10. Use of 3-nytrotirosinated fibrinogen as a therapeutical marker.

11. The use of claim 10, wherein the therapeutical treatment is selected from the following ones trombolytic, antiplatelet anticoagulant and antioxidants.

12. Use of diagnostic means capable of measuring a patient's amount of 3-nitrotyrosinated fibrinogen in any of the methods of claims 1 to 8.
